Europäisches Patentamt

(19) European Patent Office

Office européen des brevets

(11) Publication number : **0 281 411 B1**

## (12) EUROPEAN PATENT SPECIFICATION

(45) Date of publication of patent specification :
21.08.91 Bulletin 91/34

(51) Int. Cl.⁵ : **C07C 2/62**

(21) Application number : 88301904.4

(22) Date of filing : 04.03.88

(54) Alkylation process.

The file contains technical information submitted after the application was filed and not included in this specification

(30) Priority : 05.03.87 US 21985

(43) Date of publication of application :
07.09.88 Bulletin 88/36

(45) Publication of the grant of the patent :
21.08.91 Bulletin 91/34

(84) Designated Contracting States :
AT BE CH DE ES FR GB GR IT LI LU NL SE

(56) References cited :
US-A- 3 097 250
US-A- 3 105 102
US-A- 3 442 972
US-A- 3 564 073

(73) Proprietor : UOP (a New York general partnership)
25 East Algonquin Road
Des Plaines Illinois 60017-5017 (US)

(72) Inventor : O'Neil, Patrick Scott
307 Randwood Drive
Williamsville New York 14221 (US)

(74) Representative : Gore, Peter Manson et al
W.P. THOMPSON & CO. Coopers Building
Church Street
Liverpool L1 3AB (GB)

## Description

This invention pertains to the field of alkylating isoparaffinic hydrocarbons with olefinic hydrocarbons in the presence of an acid catalyst including the use of effluent refrigeration. More specifically, the present invention relates to the use of enhanced boiling surface on the inside surface area of the chiller tubes within the reaction zone.

Alkylation in the petroleum refining industry involves the chemical reaction of isoparaffinic hydrocarbons with olefinic hydrocarbons, typically isobutane with isobutylene, in the presence of a catalyst such as sulfuric or hydrofluoric acid, to product $C_8$ branched hydrocarbons such as trimethylpentanes (iso-octanes). These higher molecular weight "alkylates", as they are called, improve the anit-knock properties of motor gasoline. The increased use of unleaded fuels has considerably increased the importance of the alkylation process within the refining industry.

Alkylation has been commercially practiced for more than fifty years, and is competitive with other octane enhancement processes when there is a ready supply of isobutane and isobutylene. Generally, there are four different methods for carrying out the alkylation process today : sulfuric acid-autorefrigeration, sulfuric acid-effluent refrigeration, hydrofluoric acid-time tank, and hydrofluoric acid-tubular reactor. Of these four methods, only sulfuric acid-effluent refrigeration involves the boiling of refrigerant within a heat exchanger to cool the reaction zone. It is this method of alkylation with which the present invention is primarily directed. Reference is made to US-A-2664452 ; US-A-2906796 and US-A-2949494 which describe such an alkylation technique.

The basic chemical reaction for isobutane and isobutylene in the presence of concentrated sulfuric acid at 4.4°C to 12.8°C (40° to 55°F) is the following :

$$C_4H_{10} + C_4H_8 \rightarrow C_8H_{18} \text{ (iso-octane)}$$

Generally, in the basic process, concentrated sulfuric acid is mixed with cracked gases containing olefinic components such as propylene and isobutylene as well as propane and butane in addition to both fresh and recycled isobutane in the reaction zone, with about 40% sulfuric acid being present by volume. The fluids are not miscible, and the $C_4$ fractions float on the acid. When the reactor is vigorously agitated, the hydrocarbons break up into extremely fine droplets, and an emulsion is formed which increases the rate of reaction. After about 1 hour at 7.2°C (45°F) and 482.7 kPa (70 psia), a yield of about 15% to 20% alkylate is obtained.

Temperature is a critical variable in the alkylation reaction. The lower the temperature, the less the tendency to form undesirable side reactions involving self alkylation of the isobutane, or reaction of the acid to form alkyl sulfates. Consequently, better quality alkylate and more conversion is obtained when the reaction is carried out at the lower temperature, with the lower limit being generally about 1.7°C (about 35°F). This lower limit is primarily set by the high viscosity of the acid at that temperature and the cost of refrigeration required to remove the exothermic heat of reaction.

Since temperature is so important in the alkylation reaction and in view of the fact that the reaction is exothermic in nature, it is therefore necessary to continually remove the heat of reaction to maintain a desirable temperature of reaction. In order to do so, the sulfuric acid-effluent refrigeration technique of alkylation involves continuously passing effluent from the reactor to a settler (while the reaction is going on) to separate the effluent into a hydrocarbon phase and an acid catalyst phase. The hydrocarbon phase is then reduced in pressure thereby lowering its temperature. This cooled stream, now containing both liquid and flashed vapor created by the pressure reduction, is then passed in indirect heat exchange relationship through the reaction zone thereby removing the heat of reaction. Typically, the cooled hydrocarbon stream passes through a U-shaped tube bundle chiller which is provided within the reaction zone. As the heat of reaction is transferred to the chiller, more of the liquid hydrocarbon vaporizes inside the chiller tubes.

The large amount of vapor generated by the heat of reaction in the hydrocarbon stream is separated from the liquid portion of the stream, typically in a suction trap. The liquid goes to a fractionation step, while the vapor passes through a compressor and condenser to form a further liquid phase. This further liquid phase is then throttled to an intermediate flash tank which is at the same pressure as the suction trap, both pressures being controlled by the suction pressure on the compressor. Flashed vapors are recycled to the compressor, while the liquid phase, known commonly in the art as "effluent refrigerant" and consisting primarily of isobutane, is recycled back to the reaction zone providing additional cooling of the reaction.

While the alkylation reaction is most desirably carried out at the optimum temperature range of from 4.4°C to 7.2°C (40°F to 45°F), most commercial plants generally operate their alkylation reactors at the higher temperature of 10°C (50°F), and in some cases as high as 12.8°C (55°F) or higher, due to economical and safety factors.

More particularly, referring to the well known heat transfer equation :

$$Q = U \times A \times (T_{reactor} - T_{boiling\ fluid})$$

where Q is the heat generated by the heat of reaction in watts (BTU/hr) ; U is the overall heat transfer coefficient in units with watts/m² °k (BTU/hr)/(ft² °F)) ; A is the surface area of the chiller in m² (ft²) ; $T_{reactor}$ is the temperature of the reaction ; and $T_{boiling\ fluid}$ is the mean temperature of coolant hydrocarbon phase ; it is seen that for a given heat duty Q, there are effectively four different parameters that can theoretically be varied in order to provide the necessary heat transfer.

Of all of these parameters, increasing the surface area of the chillers involves the most costly option. Due to practical design limitations of the chiller/reactor units, commonly known in the art as "contactors", a plurality of these contactors must be provided if a substantial amount of chiller surface area is needed. Aside from the capital costs involved in providing such additional contactors, there are a number of factors which come into play which add to the disadvantages of this approach. The first effect is that additional contactors each employ an additional agitator within each respective reaction zone. This now undesirably adds mechanical energy into the reaction zone which increases the heat load of the system. Of course, the costs associated in operating these additional contactors also increases. Secondly, the reactor space velocity decreases when employing a plurality of contactors, typically in parallel. This tends to increase the production of high boiling components, such as, for example, alkylates, within the reaction effluent which undesirably elevates the boiling temperature and reduces the temperature difference available for heat transfer. Finally, inasmuch as the chiller fluid has to pass through a greater number of contactors, the tube side velocity is reduced with a concomitant reduction in the inner tube heat transfer coefficient which in turn leads to a decrease in the overall coefficient. The net result of all of the above is that increasing the chiller surface area is not the most viable alternative.

The next alternative is to attempt to decrease the temperature of the boiling fluid, i.e., the fluid passing through the chiller, so as to provide a greater temperature gradient for the required heat transfer. This approach too is disadvantageous. As noted above, the boiling fluid is obtained by partially flashing the separated hydrocarbon phase coming from the acid settler to a reduced pressure. This reduced pressure is controlled by the suction pressure of the compressor in which the vaporized hydrocarbons within the boiling fluid are ultimately passed. As would be readily apparent to one skilled in the art, the temperature of the boiling fluid entering the chiller bundle is dependent upon the suction pressure of the compressor. By reducing the suction pressure, the temperature of the boiling fluid is correspondingly reduced. However, in order to provide a boiling fluid temperature which is low enough to establish a sufficient temperature gradient, the suction pressure of the compressor would undesirably have to operate under partial vacuum. Thus, if a system were operating at a reaction temperature of about 11.1°C (about 52°F) with the boiling fluid entering the chiller at –3.9°C (25°F) at a suction pressure of 117.2 kPa (17 psia), in order to reduce the reaction temperature to 5.6°C (42°F), for example, the chiller temperature would have to enter at a temperature of –9.4°C (15°F) which would require a compressor suction pressure of 89.6 kPa (13 psia), which is below atmospheric. Moreover, due to the lower boiling fluid temperature and pressure which leads to a decrease in the vapour density, more compressor power would be required to compress the same amount of vapour.

Accordingly, attempting to accommodate the heat duty of the system by reducing the boiling fluid temperature will lead to increased power consumption or require the use of a larger compressor. Most importantly, however, it will also lead to a compressor operating at a suction pressure under vacuum. This condition could lead to the leakage of air into the system and the potentially dangerous buildup of oxygen in the hydrocarbons.

Without simply increasing the reaction temperature in order to provide an increased temperature gradient, the only other variable in the heat transfer equation is U, the overall heat transfer coefficient. This overall heat transfer coefficient is well recognized by those skilled in the art as being dependent upon the combination of the individual liquid film heat transfer coefficient on the outside of the tube which is in contact with the reaction emulsion and the individual boiling film heat transfer coefficient on the inside of the tube which is in contact with the boiling fluid.

Due to the nature of the emulsion, it has been extremely difficult to measure and/or calculate an emulsion heat transfer coefficient for the outside of the chiller tubes. By using well established relationships, it is possible, however, to calculate the heat transfer coefficient for the inner, boiling side of the tube. Having established from existing operating systems that the overall heat transfer coefficient for the chiller in such an alkylation reaction is 284 to 341 W/m² °K (50 to 60 (BTU/hr)/(ft² °F)), and after calculating the heat transfer coefficient for the boiling fluid, it is possible to extract a heat transfer coefficient for the emulsion side of the chiller. See, for example, Chen, J.C., "Industrial and Engineering Chemistry, Process Design and Development", Vol. 5 No. 3, pp. 322 (1966) for methods of calculating in-tube film coefficients.

Based upon what is conventionally known by one skilled in the art, it is generally believed that the controlling factor influencing the overall heat transfer coefficient is the emulsion heat transfer coefficient on the outside of

3

the chiller. In other words, based on calculations such as that described above, the heat transfer coefficient on the emulsion side generally is believed to be lower than the heat transfer coefficient on the inner boiling side. Consequently, if one skilled in the art were to attempt to increase the overall heat transfer coefficient, he would seek to increase the emulsion side heat transfer coefficient.

However, due to the nature of the emulsion, the prior art has generally refrained from modifying the surface of the outer tube so as to attempt to increase the outside heat transfer coefficient. Thus, a common way of increasing the coefficient would be to add fins to the outer walls of the tubes. However, such extended surfaces would be susceptible to corrosion by the acid ; fouling and clogging ; and cause a decrease in the flow. As a result, one skilled in the art has stayed away from attempting to modify the overall heat transfer coefficient as a means of providing better heat transfer.

In view of the above, the skilled art worker has found effectively no choice in many cases but to carry out the alkylation reaction at a temperature which is higher than desirable or operate the suction pressure of the compressor as low as possible, even under vacuum, in order to provide the necessary temperature gradient for the required heat transfer. Clearly, a need exists to improve this alkylation process, particularly to be able to run the reaction at the most optimum temperature without the need to run the compressor suction pressure under vacuum.

It has now been found possible to provide a sulfuric acid-effluent refrigeration alkylation process which eliminates or substantially reduces the disadvantages noted above.

According to one embodiment of the present invention there is provided a process of alkylating isoparaffinic hydrocarbons with olefinic hydrocarbons in the presence of an acid catalyst which comprises the steps of reacting isoparaffinic hydrocarbons and olefinic hydrocarbons in the presence of acid catalyst in a reaction zone to form alkylate, withdrawing a mixture of hydrocarbons with acid catalyst as effluent from the reaction zone, separating the effluent into an acid phase and a hydrocarbon phase in a first separating zone, reducing the pressure on the hydrocarbon phase to refrigerate it and vapourize volatile hydrocarbons, passing the refrigerated hydrocarbon phase in indirect heat exchange with the reaction mixture of hydrocarbons and catalyst in the reaction zone to remove exothermic heat of reaction and vapourize further volatile hydrocarbons in the hydrocarbon phase, separating the liquid portion of the hydrocarbon phase from the vapour portion thereof in a second separating zone, fractionating the liquid portion of the hydrocarbon phase to remove alkylate, passing the vapour phase material removed in the second separating zone through a compressor $a_n$d a condenser to compress and condense the vapour phase to form a liquid phase, reducing the pressure on the liquid phase to refrigerate it and vapourize volatile hydrocarbons forming an isoparaffinic hydrocarbon liquid phase and a volatile hydrocarbon vapour phase, separating the regrigerated isoparaffinic hydrocarbon liquid phase from the volatile hydrocarbon vapour phase in a third separating zone, and adding the separated refrigerated isoparaffinic hydrocarbon liquid phase as a reactant in the reaction zone, in which :

(a) the compressor is operated at a suction pressure greater than atmospheric pressure, which suction pressure is equal to the pressure within the second and third separation zones ;

(b) the reaction within the reaction zone is carried out at a temperature less than 10°C (50°F) ; and

(c) heat exchange apparatus is provided having a thermally conductive wall with an enhanced boiling surface on one side of such wall for carrying out the indirect heat exchange of the refrigerated hydrocarbon phase with the reaction mixture, wherein the refrigerated hydrocarbon phase is in contact with the said enhanced boiling surface during the indirect heat exchange.

According to another embodiment of the present invention there is provided a process of alkylating isoparaffinic hydrocarbons with olefinic hydrocarbons in the presence of an acid catalyst which comprises the steps of reacting isoparaffinic hydrocarbons and olefinic hydrocarbons in the presence of acid catalyst in a reaction zone at a first reaction temperature to form alkylate, withdrawing a mixture of hydrocarbons with acid catalyst as effluent from the reaction zone, separating the effluent into an acid phase and a hydrocarbon phase in a first separating zone, reducing the pressure on the hydrocarbon phase to refrigerate it and vapourize volatile hydrocarbons, passing the refrigerated hydrocarbon phase in indirect heat exchange with the reaction mixture of hydrocarbons and catalyst in the reaction zone to remove exothermic heat of reaction and vapourize further volatile hydrocarbons in the hydrocarbon phase, separating the liquid portion of the hydrocarbon phase from the vapour portion thereof in a second separating zone, fractionating the liquid portion of the hydrocarbon phase to remove alkylate, passing the vapour phase material removed in the second separating zone through a compressor and a condenser to compress and condense the vapour phase to form a liquid phase, reducing the pressure on the liquid phase to refrigerate it and vapourize volative hydrocarbons forming an isoparaffinic hydrocarbon liquid phase and a volatile hydrocarbon vapour phase, separating the refrigerated isoparaffinic hydrocarbon liquid phase from the volatile hydrocarbon vapour phase in a third separating zone, and adding the separated refrigerated isoparaffinic hydrocarbon liquid phase as a reactant in the reaction zone, in which

(a) heat exchange apparatus is provided having a thermally conductive wall with an enhanced boiling surface on one side of such wall for carrying out the indirect heat exchange of the refrigerated hydrocarbon phase with the reaction mixture, wherein the refirgerated hydrocarbon phase is in contact with the enhanced boiling surface during the indirect heat exchange ;

(b) the compressor is operated at a suction pressure greater than atmospheric pressure, which suction pressure is equal to the pressure within the second and third separation zones ; and

(c) the combined feed rate of isoparaffinic hydrocarbons and olefinic hydrocarbons to the reaction zone is increased by an amount equal to at least 10% of the first feed rate at a reaction temperature which is equal to or less than the said first reaction temperature.

By means of the present invention, it is now possible to operate the alkylation reaction at a temperature which is less than 10°C (50°F), preferably at a temperature within the range of from 4.4°C to 7.2°C (40°F to 45°F) and at a compressor suction pressure which is greater than the atmospheric pressure without having to alter flow rates or chiller heat transfer surface area. In fact, as a result of this invention, the capacity of the chiller can be increased for a given area and temperature gradient ; the reactor temperature can be decreased for a given capacity and heat transfer area ; and/or, the boiling fluid temperature can be increased thus increasing the compressor throughput or reduce its energy consumption.

It has been found that the above features can be preferably accomplished by utilizing an enhanced boiling surface chiller tube having a porous boiling surface on the inside of the tube. Such enhanced boiling surface tubes are well known and are available under the tradename High Flux tubes from Union Carbide Corporation, Danbury, Connecticut.

Quite surprisingly and unexpectedly, it has been found that in contradistinction to what is generally believed in the art, the controlling heat transfer coefficient in the chiller is actually on the inside of the tubes in which the boiling of the hydrocarbons takes place, and not on the outside of the tubes which is generally understood as being controlling. Thus, by providing an enhanced boiling surface in the inner tube, the overall heat transfer coefficient is surprisingly increased by a factor of as much as 2 to 2.5 or more leading to a substantial increase in the ability of the chiller to remove the heat of reaction from the reaction zone.

Such enhanced boiling surface heat exchange tubes are discussed in, for example, US-A-3384154 ; US-A-3821018 ; US-A-4064914 ; US-A-4060125 ; US-A-3906604 ; US-A-4216826 ; and US-A-3454081 all of which are incorporated herein by reference. These enhanced tubes are made in a variety of different ways which are well known to those skilled in the art. For example, such tubes may comprise annular or spiral cavities extending along the tube surface made by mechanical working of the tube. Alternatively, fins may be provided on the surface. So too, the tubes may be scored to provide ribs, grooves, a porous layer and the like.

Generally, the more efficient enhanced tubes are those having a porous layer on the boiling side of the tube which can be provided in a number of different ways well known to those skilled in the art. In one such method, as described in US-A-4064914, the porous boiling layer is bonded to one side of a thermically conductive wall. The porous boiling layer can be made of thermally conductive particles bonded together to form interconnected pores of capillary size having equivalent pore radius of less than about 0.15 mm (about 6.0 mils), and preferably less than about 0.11 mm (about 4.5 mils). As used herein, the phrase "equivalent pore radius" empirically defines a porous boiling surface layer having varied pore sized and non-uniform pore configurations in terms of an average uniform pore dimension. Such an enhanced tube containing a porous boiling layer is commercially available under the tradename High Flux tubing made by Union Carbide Corporation, Danbury, CT.

A particularly preferred characteristic of the porous surface layer is the interconnected pores of capillary size, some of which communicate with the outer surface. Liquid to be boiled enters the subsurface cavities through the outer pores and subsurface interconnecting pores, and is heated by the metal forming the walls of the cavities. At least part of the liquid is vaporized within the cavity and resulting bubbles grow against the cavity walls. A part thereof eventually emerges from the cavity through the outer pores and then rises through the liquid film over the porous layer for disengagement into the gas space over the liquid film. Additional liquid flows into the cavity from the interconnecting pores and the mechanism is continuously repeated.

By utilizing this enhanced boiling surface tubing containing a porous boiling layer, the boiling film heat transfer coefficient of the boiling fluid within the tubes is increased by a factor of about 10, typically to a value of about 2271 and even 5678 W/m² °K (about 400 and even 1,000 (BTU/hr)/(ft² °F)) or more. This is due to the fact that the heat leaving the base metal surface of the tube does not have to travel through an appreciable liquid layer before meeting a vapour-liquid surface producing evapouration. Within the porous layer, a multitude of bubbles are grown so that the heat, in order to reach a vapour-liquid boundary, need travel only through an extremely thin liquid layer having a thickness considerably less that the minute diameter of the confining pore. Vaporization of the liquid takes place entirely within the pores.

When using an enhanced boiling surface other than a porous layer, the boiling film heat transfer coefficient is typically increased by a factor of about 4 or more to a value of at least about 2271 W/m² °k (about 400 (BTU/hr) (ft² °F)).

The utilization of this enhanced boiling surface tubing not only increases the overall heat transfer coefficient thereby increasing the available capacity of the chiller but, moreover, provides yet additional advantages.

Firstly, the use of this enhanced tubing permits the temperature of the boiling fluid within the chiller to enter at a higher temperature. Generally, the temperature of the boiling fluid can be increased, at a given temperature of reaction, by an amount of 5°C to 6.7°C (9° to 12°F), providing a temperature gradient between the boiling fluid and the reaction mixture of from 5.6°C to 11.1°C (10° to 20°F), preferably 5.6°C to 8.3°C (10° to 15°F). This increased boiling fluid temperature also leads to an increase in the boiling fluid pressure. This, in turn, reduces the energy consumption of the compressor. In general, a 10 degree increase in boiling fluid temperature reduces the power consumption of the compressor by about 12%. Finally, the increase in boiling fluid pressure enables the operation of the compressor of the compressor suction to be greater than atmospheric, preferably from 0 to 48.3 kPa gauge (0 to 7 psig), and most preferably in the range of from about 13.8 to 27.6 kPa (about 2 to 4 psig).

Once a portion of the extra heat transfer capability is utilized by increasing the temperature of the boiling fluid to ensure that the compressor suction is greater than atmospheric pressure, there is still generally yet additional heat transfer capability left to be effectively utilized. This extra cooling capacity can all be used to cool the reaction temperature to its optimum temperature or, alternatively, by keeping the reaction temperature constant, it can be used to increase the feed rate of the reactants to the reaction zone thereby producing more alkylate product. As still another alternative, the extra cooling capacity may be distributed between reducing the reaction temperature and increasing the throughput of the system.

By increasing the overall heat transfer coefficient, the temperature of the reaction can be operated at the optimum temperature. The effect of reactor temperature on alkylate quality is quite significant. For a 5.6°C (10 degree F) reduction in temperature, the octane number (RON) of the alkylate is increased by about 0.5 to 0.72 points. In addition, the lower temperature reduces the consumption of acid as well. Each 5.6°C (10 degree F) reduction in temperature reduces acid consumption of 9.59 grams of sulfuric acid per litre of alkylate product (0.08 lbs. of sulfuric acid per gallon of alkylate product). Still further, a decrease in reaction temperature would reduce the tendency for undesirable side reactions, and increase the amount and quality of alkylate product. Generally, the reaction temperature can be reduced to a temperature less than 10°C (50°F), preferably to a temperature of 4.4°C to 7.2°C (40° to 45°F).

Although temperature of reaction is quite important, there may be times in which an increase in product output capacity is also important. As a result of the increased overall heat transfer coefficient and an increase in the boiling fluid temperature and pressure, the capacity of the chiller-compressor system can be increased by at least about 10%. Thus, at constant compressor power, as a result of the higher vapor temperature entering the compressor which in turn leads to a higher vapor density, more vapor is compressed and condensed on a weight basis providing for improved chiller capacity. By operating the suction pressure of the compressor at maximum, typically about 6 psig, and carrying out the reaction at a maximum temperature of 12.8°C to 14.4°C (55 to 58°F), the increase in the capacity of the system is about 15 to 20%.

The present invention will now be further described with reference to and as illustrated in, but in no manner limited to, the accompanying drawings, in which :

Figure 1 is a schematic flow diagram of one embodiment of the present invention showing an acid-effluent refrigeration alkylation process utilizing an enhanced chiller tube within the contactor ; and

Figure 2 is a cross-section of one of the chiller tubes used in the contactor of Figure 1.

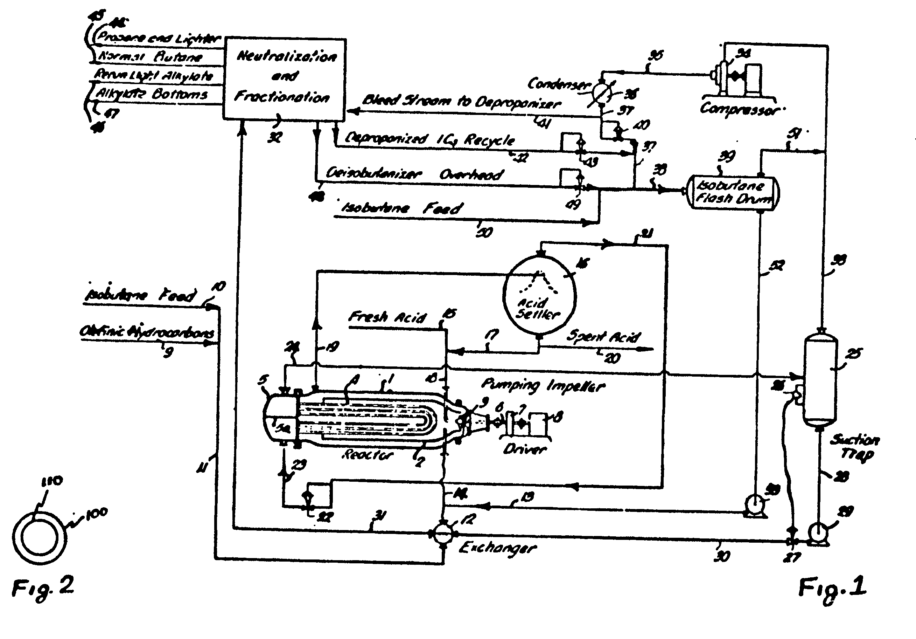

Referring to Figure 1, at 1 is a reactor shell equipped with an open-ended circulating tube 2. In one end of the circulating tube is a propeller or pump impeller 3 and within the circulating tube is a chiller consisting of a tube bundle 4 having internal enhanced boiling surface provided with a distributing head 5 which encloses one end of the reactor. The impeller is mounted on a shaft 6 rotated through a reduction gear 7 by any suitable means such as an electric motor or steam turbine shown diagrammatically as 8.

Circulation within the reactor is established by the impeller through the annular space between the shell and circulating tube 2 over the cooling tubes 4 and back to the impeller.

Olefinic hydrocarbons and isoparaffinic hydrocarbons, such as, for example, isobutane, are introduced to the system through lines 9 and 10, respectively, being combined in feed line 11 prior to passage through heat exchanger 12. Recycled isobutane returned through line 13 is introduced into the feed in line 14. Fresh acid, such as, for example, sulfuric acid, is fed to the reactor through line 15 and recycle acid from settler 16 is returned through line 17. The hydrocarbons supplied through lines 9 and 10 mixed with recycled isobutane added through line 13 are mixed in the reactor with the acid catalyst introduced through lines 15, 17 and 18.

Alkylation of the isoparaffinic hydrocarbons by the olefinic hydrocarbons takes place in the reactor 1, while the mixture is being rapidly circulated and agitated by impeller 3 insuring a thorough and intimate mixture of the hydrocarbons with acid catalyst. The mixture of hydrocarbons and acid is discharged from the reactor through line 19 passing to the acid settler 16 where it is permitted to separate into a heavier acid phase and a hydrocarbon phase. The acid phase is recycled to the suction side of the pumping side of impeller 3 of the reactor through line 17, while a portion of the acid separated in the settler may be discarded through the spent acid discharge line 20 to maintain a proper balance and proportioning of catalyst and reactants in the system.

The hydrocarbon phase separated in the settler is discharged from the top of the settler through line 21, and pressure upon these hydrocarbons is reduced by throttling at valve 22, after which the liquid/vapor mixture is passed immediately through line 23 to the distributing head 5 of the reactor. The head 5 is divided by a partition 5a which causes the coolant to pass through the heat exchange elements or enhanced tube bundle 4, then into the opposite side of the distributing head and out through line 24. The temperature of the reaction will generally be less than 70°C (50°F), and preferably in the range of 4.4°C to 10°C (40°F to 50°F), and most preferably in the range of 4.4°C to 7.2°C (40°F to 45°F).

Upon passing valve 22, pressure on the hydrocarbon phase of the effluent is reduced to the order of 0 to 69 kPa gauge (0 psig to 10 psig), preferably 13.8 to 27.6 kPa (2 to 4 psig), causing a considerable portion of the lighter components of the effluent to vapourize and resulting in the cooling of the entire hydrocarbon effluent mixture. Depending upon the pressure established within the tube bundle 4 of the reactor, the temperature of the hydrocarbon effluent phase will be reduced to be in the range of from about 8.3°C to 13.9°C (about 15° to 25°F) by the reduction of pressure. This chilled effluent, which is a mixture of liquid and vapour, while passing through the enhanced chiller tubes 4 of the reactor absorbs the exothermic heat of alkylation reaction by indirect heat exchange resulting in vapourization of additional lighter components of the effluent.

Upon leaving the chiller tubes 4 of the reactor, the partially vaporized effluent passes from the opposite side of the circulating head through line 24 to suction trap 25 where the vapor and liquid portions of the effluent are separated. A liquid level control 26 manipulating valve 27 regulates the discharge of the liquid phase from the suction trap through line 28. This liquid is returned by pump 29 through line 30 to heat exchanger 12 where it is is brought in heat exchange relation with the incoming feed stock. From the heat exchanger, the liquid passes through line 31 to the neutralization and fractionation steps diagrammatically shown as 32.

The vapors separated from the effluent in suction trap 25 pass out through line 33 to compressor 34 from which they are discharged through line 35 to condenser 36 where they are totally condensed. A portion of the condensate from condenser 36 is directed through lines 37 and 38 to isobutane flash drum 39 which is operated at the same pressure as suction trap 25, both pressures being controlled by the suction pressure on compressor 34 which, in accordance with the present invention operates at a pressure which is greater than atmospheric and is equal to the presssure of the hydrocarbon phase after passing valve 22. Interposed in line 37 is a pressure reducing valve 40 which holds sufficient back pressure on the condenser 36 to make possible total condensation of the hydrocarbons. Liquid hydrocarbons passing through valve 40 are thereby reduced in pressure causing partial vaporization and chilling of the hydrocarbons prior to their introduction into flash drum 39.

When propane is a component of any of the feed streams, a portion of the condensate withdrawn through line 37 is diverted through line 41 to the depropanizer of the fractionation section 32. After depropanization, this stream is returned to the system through line 42, pressure reducing valve 43 and lines 37 and 38 to the isobutane flash drum 39. Back pressure valve 43 in line 42 functions in the same manner as reducing valve 40 described above.

The liquid hydrocarbons withdrawn from suction trap 25 and passed to fractionation are there separated into streams of propane, normal butane, light alkylate and alkylate bottoms. The product streams are normally removed from the system through lines 44, 45, 46 and 47, respectively. The isobutane stream taken overhead from the deisobutanizer tower is recycled through line 48, reduction valve 49 and line 38 to the isobutane flash drum from which it is directed to the reaction zone in reactor 1. Fresh isobutane feed to the system may also be brought in either through line 10 or through line 50 which connects through line 38 to the isobutane flash drum. All of the streams entering the isobutane drum 39 are subjected to reduced pressure established by the suction of the compresssor and are thereby self-refrigerated. The vapors evolved in the isobutane flash drum by this self-refrigeration are passed through line 51 to the compressor, while the chilled liquid from the drum, principally isobutane, is directed through line 52 to pump 53 and then through lines 13 and 14 to the reactor.

Figure 2 depicts a cross section of the enhanced chiller tubes in which outer surface 100 contacts the reaction mixture and inner surface 110, containing the preferred enhancement of a porous layer, contacts the boiling fluid.

The preferred enhanced tube for use in the alkylation contactor ranges in diameter from 19.1 mm to 31.8 mm (0.75 to 1.25 inch), with 25.4 mm (1.0 inch) being most common. The tube wall thickness ranges in thickness from 2.0 mm to 3.8 mm (0.08 to 0.15 inch), with 2.5 mm (0.10 inch) being preferred. Although the tube material may be comprised of any thermally conductive material, ferrous or stainless alloy is commonly used, the preferred material being ordinary carbon steel.

## EXAMPLE

A comparison is made of two process conditions for the sulfuric acid alkylation reaction, one using contactor bundles with High Flux tubing, and the other with conventional tubes. In this example, a typical $1.59 \times 10^6$ litres per stream per day (LPSD), equivalent to 10,000 barrel per stream per day (BPSD) in a plant having four contactors operating in parallel with two settling tanks are employed. Each settler is fed by two contactors. The total olefin and isobutane feed flow is $10.49 \times 10^6$ LPSD (66,000 BPSD), while the refrigeration compressor approximately 64080 kg.cal/min (6,000 hp) has a suction pressure fixed at 117.2 kPa (17 psia), to avoid vacuum operation.

The details of the comparison are shown in Table I below. Each of the four contactors has a volume of 49209 litres (13,000 gallons), and a chiller bundle with a heat transfer area of 790 $m^2$ (8,500 $ft^2$). The volume of each of the acid settler is 348248 litres (92,000 gallons). A high rate of internal circulation of the emulsion is maintained by impellers with a total power consumption of 12816 kg.cal/min (1,200 hp). The total heat of reaction, including energy imparted to the fluid, is 12 million W (41 million BTU/hr). Hydrocarbon from the settler is throttled and fed to the boiling side of the chiller. To remove the heat of reaction, the boiling flow, at 244940 kg (540,000 lb/hr), is approximately 50% vapourized. The boiling stream containing about 18% $C_8$ alkylate, enters at –3.9°C (25°F) and exists at 1.7°C (35°F). In order to maintain enough temperature difference to transfer the heat of reaction, consistent with an overall heat transfer coefficient of about 568 W/$m^2$ °K (about 100 (BTU/hr)/($ft^2$ °F)) when utilizing High Flux tubing, and 284 W/$m^2$ °K (50 (BTU/hr)/($ft^2$ °F)) when using a bare tube bundle, the reactor temperature must be maintained at 6.1°C (43°F) and 12.8°C (55°F), respertively.

As seen in Table I, the relative octane number (RON) increases about 0.7 points, due to the lower reactor temperature, for the case using the High Flux tube bundle. In the examples, the reactor volume per BPSD of alkylate product is that typically used in commercial practice, namely 15 litres to 17 litres (4 to 4.5 gallons).

Case 3 in Table I illustrates the effect of attempting to lower the reactor temperature by adding more contactors. Since the overall coefficient for the High Flux tube bundle is about two times higher than for the bare tubes, it is assumed that doubling the number of bare tube contactors would reduce the reactor temperature to 6.1°C (43°F), the value achieved by the High Flux tubes. In reality, several additional effects occur which tend to make the necessary area increase even greater than two-fold. The first effect is that an additional 12816 kg.cal/min (1200 hp) mechanical energy enters the circulating fluid from the impellors and must be removed. This increases the heat load to about 17300000 W (about 44 million BTU/hr). Since the boiling flow is fixed, more must vapourize which increases the outlet temperature. The second effect is that the reactor space velocity decreases which tends to increase the conversion or yield of $C_8$ alkylate, and thus the boiling temperature of the mixture. A third effect is that the total boiling flow of 244940 kg (540,000 lb/hr) is now distributed over eight or more bundles rather than four, which at least halves the tube side velocity and reduces the tube side heat transfer coefficient. The net result is that considerably more than double the number of reactors must be added which is not feasible in an existing plant.

TABLE I

EXAMPLE OF IMPROVED 15.9x10⁶ LPSD (10,000 BPSD)

ALKYLATION PROCESS USING HIGH FLUX

| ITEM | CASE 1 HIGH FLUX | CASE 2 CONVENTIONAL TUBES | CASE 3 CONV'T. TUBES REACTOR AT TEMP-CASE 1 | REMARKS CASE 3 |
|---|---|---|---|---|
| Alkylate Capacity (LPSD (BPSD | $1.59 \times 10^6$ 10,000 | $1.50 \times 10^6$ 10,000 | $1.59 \times 10^6$ ) 10,000 ) | Fixed |
| Total Feed Flow (LPSD (BPSD | $10.49 \times 10^6$ 66,000 | $10.49 \times 10^6$ 66,000 | $10.49 \times 10^6$ ) 66,000 ) | Fixed |
| No. Chillers in Parallel | 4 | 4 | 8+ | High |
| Volume of each Chiller (Gal (ℓ | 13,000 49,209 | 13,000 49,209 | 13,000 ) 49,209 ) | Fixed |
| Area (Heat Transfer/Chiller) ($m^2$ ($ft^2$ | 790 8,500 | 790 8,500 | 790 ) 8,500 ) | Fixed |
| No. Acid Settlers | 2 | 2 | 4 | Doubled |
| Volume of Settler (Gal. (ℓ | 92,000 348,248 | 92,000 348,248 | 92,000 ) 348,248 ) | Fixed |
| Space Velocity, BPSD Feed/ Barrels Reactor Volume | 53 | 53 | 26 | Equals (42 gal/bbl) (66,000)/(13,000) x(no. chillers) |

TABLE I (Contd.)

EXAMPLE OF IMPROVED 15.9x10⁶ LPSD (10,000 BPSD)

ALKYLATION PROCESS USING HIGH FLUX

| ITEM | CASE 1 HIGH FLUX | CASE 2 CONVENTIONAL TUBES | CASE 3 CONV'T. TUBES REACTOR AT TEMP-CASE 1 | REMARKS CASE 3 |
|---|---|---|---|---|
| % Alkylate in Boiling Feed | 18 | 18 | 20%+ | Because space velocity decreased |
| Total Flow to Boiling Side Number/hr. | 540,000 | 540,000 | 540,000 | Fixed |
| Amount Vaporized, Boiling Side % | 50 | 50 | Approx. 60+ | Increases because heat load increased |
| Total Contactors Heat Duty MMBTU (mm W-sec | 43228 | 43228 | 46391+ | Duty increased because of increased agitator power |
| (mm BTU | 41.0 | 41.0 | 44+ | |
| Agitator Power (Total) (HP | 1,200 | 1,200 | 2,400+ | Increased because number of reactors increased |
| (kg cal/min | 12816 | 12816 | 25632+ | |
| Boiling Inlet Temp. (°F | 25 | 25 | 28+ | Increased because alkylate increased |
| (°C | -3.9 | -3.9 | -2.2+ | |
| Boiling Outlet Temp (°F | 35 | 35 | 40+ | Increased because alkylate and amount of vapor increased |
| (°C | 1.7 | 1.7 | 4.4+ | |

EP 0 281 411 B1

TABLE I (Contd.)

EXAMPLE OF IMPROVED $15.9 \times 10^6$ LPSD (10,000 BPSD)

ALKYLATION PROCESS USING HIGH FLUX

| ITEM | CASE 1<br><br>HIGH FLUX | CASE 2<br>CONVENTIONAL<br>TUBES | CASE 3<br>CONV'T. TUBES<br>REACTOR AT<br>TEMP-CASE 1 | REMARKS CASE 3 |
|---|---|---|---|---|
| Compressor Suction (PSIA<br>(kPa | 17<br>117 | 17<br>117 | 17<br>117 | Vacuum operation<br>avoided |
| Reactor Temp. ($^{\circ}$F<br>($^{\circ}$C | 43<br>6.1 | 55<br>12.8 | 43<br>6.1 | Not feasible |
| Mean T ($^{\circ}$F<br>($^{\circ}$C | 12.3<br>6.8 | 24.6<br>13.7 | <12<br><6.7 | Decreased because<br>of alk. and % vapour<br>increase |
| Overall Coeff., (Design) | 98 | 49 | <49 | Because of decreased<br>boiling flow/chiller |
| Relative Octane Number<br>Increase | 0.72 | 0 | 0.72 | Only if reactor is<br>at 6.1$^{\circ}$C (43$^{\circ}$F) |
| Reactor volume/159 LPSD (BPSD)<br>Used | 5.2 | 5.2 | 10.4+ | At least doubled |
| Reactor Volume/159 LPSD (BPSD)<br>Trade Practice | 4.0-4.5 | 4.0-4.5 | | |
| Reactor Area/Radio ($cm^2$/LPSD<br>($ft^2$/BPSD | 0.58<br>3.4 | 0.58<br>3.4 | 1.17+<br>6.8+ | |
| Residence Time, Sec. | 1,630 | 1,630 | 3,260+ | |

## Claims

1. A process of alkylating isoparaffinic hydrocarbons with olefinic hydrocarbons in the presence of an acid catalyst which comprises the steps of reacting isoparaffinic hydrocarbons and olefinic hydrocarbons in the presence of acid catalyst in a reaction zone (2) to form alkylate, withdrawing a mixture of hydrocarbons with acid catalyst as effluent from the reaction zone (2), separating the effluent into an acid phase and a hydrocarbon phase in a first separating zone (16), reducing the pressure on the hydrocarbon phase to refrigerate it and vaporise volatile hydrocarbons, passing the refrigerated hydrocarbon phase in indirect heat exchange with the reaction mixture of hydrocarbons and catalyst in the reaction zone to remove exothermic heat of reaction and vaporise further volative hydrocarbons in the hydrocarbon phase, separating the liquid portion of the hydrocarbon phase from the vapour portion thereof in a second separating zone (25), fractionating the liquid portion of the hydrocarbon phase to remove alkylate, passing the vapour phase material removed in the second separating zone through a compressor (34) and a condenser (36) to compress and condense the vapour phase to form a liquid phase, reducing the pressure on the liquid phase to refrigerate it and vaporise volatile hydrocarbons forming an isoparaffinic hydrocarbon liquid phase and a volatile hydrocarbon vapour phase, separating the refrigerated isoparaffinic hydrocarbon liquid phase from the volatile hydrocarbon vapour phase in a third separating zone (39), and adding the separated refrigerated isoparaffinic hydrocarbon liquid phase as a reactant in the reaction zone, characterised in that

    (a) the compressor (34) is operated at a suction pressure greater than atmospheric pressure, which suction pressure is equal to the pressure within the second and third separation zones ;

    (b) the reaction within the reaction zone is carried out at a temperature less than 10°C (50°F) ; and

    (c) heat exchange apparatus (4) is provided having a thermally conductive wall with an enhanced boiling surface on one side of such wall for carrying out the indirect heat exchange of the refrigerated hydrocarbon phase with the reaction mixture, wherein the refrigerated hydrocarbon phase is in contact with the said enhanced boiling surface during the indirect heat exchange.

2. A process according to claim 1, wherein the reaction temperature is in the range of from 4.4°C to 7.2°C (40°F to 45°F).

3. A process of alkylating isoparaffinic hydrocarbons with olefinic hydrocarbons in the presence of an acid catalyst which comprises the steps of reacting isoparaffinic hydrocarbons and olefinic hydrocarbons in the presence of acid catalyst in a reaction zone (2) at a first reaction temperature to form alkylate, withdrawing a mixture of hydrocarbons with acid catalyst as effluent from the reaction zone, separating the effluent into an acid phase and a hydrocarbon phase in a first separating zone (16), reducing the pressure on the hydrocarbon phase to refrigerate it and vaporise volatile hydrocarbons, passing the refrigerated hydrocarbon phase in indirect heat exchange with the reaction mixture of hydrocarbons and catalyst in the reaction zone to remove exothermic heat of reaction and vaporise further volatile hydrocarbons in the hydrocarbon phase, separating the liquid portion of the hydrocarbon phase from the vapour portion thereof in a second separating zone (25), fractionating the liquid portion of the hydrocarbon phase to remove alkylate, passing the vapour phase material removed in the second separating zone through a compressor (34) and a condenser (36) to compress and condense the vapour phase to form a liquid phase, reducing the pressure on the liquid phase to refrigerate it and vaporize volatile hydrocarbons forming an isoparaffinic hydrocarbon liquid phase and a volatile hydrocarbon vapour phase, separating the refrigerated isoparaffinic hydrocarbon liquid phase from the volatile hydrocarbon vapour phase in a third separating zone, and adding the separated refrigerated isoparaffinic hydrocarbon liquid phase as a reactant in the reaction zone, characterised in that,

    (a) heat exchange apparatus (4) is provided having a thermally conductive wall with an enhanced boiling surface on one side of such wall for carrying out the indirect heat exchange of the refrigerated hydrocarbon phase with the reaction mixture, wherein the refrigerated hydrocarbon phase is in contact with the enhanced boiling surface during the indirect heat exchange ;

    (b) the compressor (34) is operated at a suction pressure greater than atmospheric pressure, which suction pressure is equal to the pressure within the second and third separation zones ; and

    (c) the combined feed rate of isoparaffinic hydrocarbons and olefinic hydrocarbons to the reaction zone is increased by an amount equal to at least 10% of the first feed rate at a reaction temperature which is equal to or less than the said first reaction temperature.

4. A process according to any of claims 1 to 3, wherein the enhanced boiling surface is a porous boiling layer.

5. A process according to claim 4, wherein the porous boiling layer is constructed of thermally conductive particles bonded together to form interconnected pores of capillary size having an equivalent pore radius of less than about 0.15 mm (about 6.0 mils).

6. A process according to claim 4, wherein the porous boiling layer is formed by mechanically working the wall surface.

7. A process according to any of claims 1 to 6, wherein the enhanced boiling surface provides a boiling film heat transfer coefficient of greater than 2271 W/m² K (400 (BRU/hr) (ft² °F)).

8. A process according to claim 7, wherein the porous boiling layer provides a boiling film heat transfer coefficient of greater than 5678 W/m² K (1000 (BTU/hr) (ft² °F)).

9. A process according to any of claims 1 to 8, wherein the suction pressure of the comperssor is in the range of from 0 to 48.3 kPa gauge (0 to 7 psig).

10. A process according to claim 9, wherein the suction pressure of the compressor is in the range of from 73.8 to 27.6 kPa gauge (2 to 4 psig).

11. A process according to any of claims 1 to 10, wherein the difference in temperature between the refrigerated hydrocarbon phase and the reaction mixture during the indirect heat exchange is in the range of from 5.6°C to 11.1°C (10° to 20°F).

12. A process according to claim 11, wherein the temperature difference is in the range of from 5.6°C to 8.3°C (10° to 15°F).


## Patentansprüche

1. Verfahren zur Alkylierung isoparaffinischer Kohlenwasserstoffe mit olefinischen Kohlenwasserstoffen in Gegenwart eines Säurekatalysators, umfassend die Stufen : Umsetzen isoparaffinischer Kohlenwasserstoffe und olefinischer Kohlenwasserstoffe in Gegenwart eines Säurekatalysators in einer Reaktionszone (2) zur Alkylatbildung, Abziehen eines Gemisches von Kohlenwasserstoffen mit Säurekatalysator als Abstrom aus der Reaktionszone (2), Trennung des Abstroms in eine saure Phase und eine Kohlenwasserstoffphase in einer ersten Trennzone (16), Druckverminderung an der Kohlenwasserstoffphase, um sie abzukühlen und flüchtige Kohlenwasserstoffe zu verdampfen, Überführen der gekühlten Kohlenwasserstoffphase in einen indirekten Wärmeaustausch mit dem Reaktionsgemisch aus Kohlenwasserstoffen und Katalysator in der Reaktionszone, um exotherme Wärme abzuführen und weitere flüchtige Kohlenwasserstoffe in der Kohlenwasserstoffphase zu verdampfen, Trennung des flüssigen Anteils der Kohlenwasserstoffphase von deren dampfförmigem Anteil in einer zweiten Trennzone (25), Fraktionieren des flüssigen Anteils der Kohlenwasserstoffphase zwecks Entfernung von Alkylat. Leiten des in der zweiten Trennzone entfernten Dampfphasenmaterials durch einen Kompressor (34) und einen Kondensator (36), um die Dampfphase zwecks Bildung einer Flüssigphase zu komprimieren und kondensieren, Druckverminderung an der Flüssigphase, um sie zu kühlen und flüchtige Kohlenwasserstoffe zu verdampfen und eine Flüssigphase aus isoparaffinischen Kohlenwasserstoffen und eine Dampfphase aus flüchtigen Kohlenwasserstoffen zu bilden, Abtrennung der gekühlten Flüssigphase aus isoparaffinischen Kohlenwasserstoffen von der Dampfphase aus flüchtigen Kohlenwasserstoffen in einer dritten Trennzone (39) und Zusetzen der abgetrennten gekühlten Flüssigphase aus isoparaffinischen Kohlenwasserstoffen als Reaktant in die Reaktionszone. dadurch gekennzeichnet, daß man

(a) den Kompressor (34) bei einem den Atmosphärendruck übersteigenden Saugdruck betreibt, wobei der Saugdruck gleich dem Druck innerhalb der zweiten und dritten Trennzone ist ;

(b) die Reaktion innerhalb der Reaktionszone bei einer Temperatur von weniger als 10°C (50°F) durchführt; und

(c) eine Wärmeaustauschvorrichtung (4) vorsieht. die eine wärmeleitende Wand mit einer vergrößerten Siedeoberfläche an einer Seite der Wand besitzt, um den indirekten Wärmeaustausch der gekühlten Kohlenwasserstoffphase mit dem Reaktionsgemisch durchzuführen, worin die gekühlte Kohlenwasserstoffphase mit der vergrößerten Siedeoberfläche während des indirekten Wärmeaustausches in Berührung steht.

2. Verfahren nach Anspruch 1, dadurch gekennzeichnet, daß die Reaktionstemperatur im Bereich von 4,4°C bis 7,2°C (40°F bis 45°F) liegt.

3. Verfahren zur Alkylierung isoparaffinischer Kohlenwasserstoffe mit olefinischen Kohlenwasserstoffen in Gegenwart eines Säurekatalysators. umfassend die Stufen : Umsetzen isoparaffinischer Kohlenwasserstoffe und olefinischer Kohlenwasserstoffe in Gegenwart eines Säurekatalysators in einer Reaktionszone (2) bei einer ersten Reaktionstemperatur zur Alkylatbildung, Abziehen eines Gemisches von Kohlenwasserstoffen mit Säurekatalysator als Abstrom aus der Reaktionszone, Trennung des Abstroms in eine saure Phase und eine Kohlenwasserstoffphase in einer ersten Trennzone (16), Druckverminderung an der Kohlenwasserstoffphase, um sie abzukühlen und flüchtige Kohlenwasserstoffe zu verdampfen, Überführen der gekühlten Kohlenwasserstoffphase In einen indirekten Wärmeaustausch mit dem Reaktionsgemisch aus Kohlenwasserstoffen und Katalysator in der Reaktionszone, um exotherme Wärme abzuführen und weitere flüchtige Kohlenwasserstoffe in der Kohlenwasserstoffphase zu verdampfen, Trennung des flüssigen Anteils der Kohlenwasserstoffphase

13

von deren dampfförmigem Anteil in einer zweiten Trennzone (25), Fraktionieren des flüssigen Anteils der kohlenwasserstoffphase zwecks Entfernung von Alkylat, Leiten des in der zweiten Trennzone entfernten Dampfphasenmaterials durch einen Kompressor (34) und einen Kondensator (36), um die Dampfphase zwecks Bildung einer Flüssigphase zu komprimieren und kondensieren, Druckverminderung an der Flüssigphase, um sie zu kühlen und flüchtige Kohlenwasserstoffe zu verdampfen und eine Flüssigphase aus isoparaffinischen Kohlenwasserstoffen und eine Dampfphase aus flüchtigen Kohlenwasserstoffen zu bilden, Abtrennung der gekühlten Flüssigphase aus isoparaffinischen Kohlenwasserstoffen von der Dampfphase aus flüchtigen Kohlenwasserstoffen in einer dritten Trennzone und Zusetzen der abgetrennten gekühlten Flüssigphase aus isoparaffinischen Kohlenwasserstoffen als Reaktant in die Reaktionszone. dadurch gekennzeichnet, daß man

(a) eine Wärmeaustauschvorrichtung (4) vorsieht, die eine wärmeleitende Wand mit einer vergrößerten Siedeoberfläche an einer Seite der Wand besitzt, um den indirekten Wärmeaustausch der gekühlten Kohlenwasserstoffphase mit dem Reaktionsgemisch durchzuführen, worin die gekühlte Kohlenwasserstoffphase mit der vergrößerten Siedeoberfläche während des indirekten Wärmeaustausches in Berührung steht ;

(b) den Kompressor (34) bei einem den Atmosphärendruck übersteigenden Saugdruck betreibt, wobei der Saugdruck gleich dem Druck innerhalb der zweiten und dritten Trennzone ist ;

(c) die kombinierte Beschickungsgeschwindigkeit isoparaffinischer Kohlenwasserstoffe und olefinischer Kohlenwasserstoffe zur Reaktionszone in einem Ausmaß von gleich wenigstens 10% der ersten Beschickungsgeschwindigkeit bei einer Reaktionstemperatur, die gleich oder geringer als die erste Reaktionsgeschwindigkeit ist, erhöht.

4. Verfahren nach einem der Ansprüche 1 bis 3, dadurch gekennzeichnet, daß die vergrößerte Siedeoberfläche eine poröse Siedeschicht ist.

5. Verfahren nach Anspruch 4, dadurch gekennzeichnet, daß die poröse Siedeschicht aus wärmeleitenden Teilchen besteht, die zur Bildung von miteinander verbundenen Poren von Kapillargröße mit einem äquivalenten Porenradius von weniger als etwa 0,15 mm (etwa 6,0 mils) miteinander verbunden sind.

6. Verfahren nach Anspruch 4, dadurch gekennzeichnet, daß die poröse Siedeschicht durch mechanische Bearbeitung der Wandoberfläche gebildet ist.

7. Verfahren nach einem der Ansprüche 1 bis 6, dadurch gekennzeichnet, daß die vergrößerte Siedeoberfläcke einen Siedefilm-Wärmeübergangskoeffizienten von mehr als 2271 W/m² K (400 (BRU/hr) (ft² °F)) schafft.

8. Verfahren nach Anspruch 7, dadurch gekennzeichnet, daß die poröse Siedeschicht einen Sidefilm-Wärmeübergangskoeffizienten von mehr als 5678 W/m² K (1000 (BTU/hr) (ft² °F)) schafft.

9. Verfahren nach einem der Ansprüche 1 bis 8, dadurch gekennzeichnet, daß der Saugdruck des Kompressors im Bereich von 0 bis 48,3 kPa (0 bis 7 psig) liegt.

10. Verfahren nach Anspruch 9, dadurch gekennzeichnet, daß der Saugdruck des Kompressors im Bereich von 73,8 bis 27,6 kPa (2 bis 4 psig) liegt.

11. Verfahren nach einem der Ansprüche 1 bis 10, dadurch gekennzeichnet, daß der Temperaturunterschied zwischen der gekühlten Kohlenwasserstoffphase und dem Reaktionsgemisch während des indirekten Wärmeaustausches im Bereich von 5,6°C bis 11,1°C (10° bis 20°F) liegt.

12. Verfahren nach Anspruch 11, dadurch gekennzeichnet, daß der Temperaturunterschied im Bereich von 5,6°C bis 8,3°C (10° bis 15°F) liegt.


## Revendications

1. Procédé d'alkylation d'hydrocarbures isoparaffiniques avec des hydrocarbures oléfiniques en présence d'un catalyseur acide, comprenant les étapes de mise en réaction d'hydrocarbures isoparaffiniques et d'hydrocarbures oléfiniques, en présence d'un catalyseur acide, dans une zone de réaction (2) pour la formation d'alkylat, le soutirage hors de la zone de réaction (2) d'un mélange d'hydrocarbures et de catalyseur acide en tant qu'effluent, de séparation de l'effluent en une phase acide et une phase d'hydrocarbures dans une première zone de séparation (16), de réduction de la pression exercée sur la phase d'hydrocarbures afin de la refroidir et de vaporiser les hydrocarbures volatils, de passage dans la zone de réaction de la phase d'hydrocarbures refroidie, en échange thermique indirect avec le mélange réactionnel d'hydrocarbures et de catalyseur, pour éliminer la chaleur de réaction exothermique et vaporiser de nouveaux hydrocarbures volatils dans la phase d'hydrocarbures, de séparation de la partie liquide de la phase d'hydrocarbures d'avec la partie vapeur de celle-ci dans une seconde zone de séparation (25), de fractionnement de la partie liquide de la phase d'hydrocarbures pour la séparation d'alkylat, de passage à travers un compresseur (34) et un condenseur (36) du produit en phase vapeur extrait dans la seconde zone de séparation, pour compresser et condenser la phase vapeur afin de former une phase liquide, de réduction de la pression exercée sur la phase liquide afin de la refroidir et de vaporiser des hydrocarbures volatils, pour la formation d'une phase liquide d'hydrocarbures isoparaffiniques et d'une phase vapeur d'hydrocarbures volatils, de séparation de la phase liquide d'hydrocarbures isoparaffiniques refroidie d'avec la phase vapeur d'hydrocarbures volatils dans une troisième zone de séparation (39), et d'addition de la phase liquide d'hydrocarbures isoparaffiniques refroidie séparée, en tant que corps réagissant dans la zone de réaction, caractérisé en ce que

(a) on fait fonctionner le compresseur (34) sous une pression d'aspiration supérieure à la pression atmosphérique, laquelle pression d'aspiration est égale à la pression dans les seconde et troisième zones de séparation ;

(b) la réaction dans la zone de réaction est effectuée à une température inférieure à 10°C (50°F) ; et

(c) l'appareil d'échange thermique (4) est muni d'une paroi conductrice de la chaleur avec une surface d'ébullition améliorée sur un côté de cette paroi pour effectuer l'échange thermique indirect entre la phase d'hydrocarbures refroidie et le mélange réactionnel, la phase d'hydrocarbures refroidie étant en contact avec ladite surface d'ébulliton améliorée, au cours de l'échange thermique indirect.

2. Procédé selon la revendication 1, dans lequel la température de la réaction est dans la plage de 4,4 à 7,2°C (40-45°F).

3. Procédé d'alkylation d'hydrocarbures isoparaffiniques avec des hydrocarbures oléfiniques, en présence d'un catalyseur acide, qui comprend les étapes de mise en réaction d'hydrocarbures isoparaffiniques et d'hydrocarbures oléfiniques, en présence d'un catalyseur acide, dans une zone de réaction (2) à une première température de réaction pour la formation d'alkylat, de soutirage hors de la zone de réaction d'un mélange d'hydrocarbures et de catalyseur acide en tant qu'effluent, de séparation de l'effluent en une phase acide et une phase d'hydrocarbures dans une première zone de séparation (16), de réduction de la pression exercée sur la phase d'hydrocarbures afin de la refroidir et de vaporiser les hydrocarbures volatils, de passage dans la zone de réaction de la phase d'hydrocarbures refroidie, en échange thermique indirect avec le mélange réactionnel d'hydrocarbures et de catalyseur, pour éliminer la chaleur de réaction exothermique et vaporiser de nouveaux hydrocarbures volatils dans la phase d'hydrocarbures, de séparation de la partie liquide de la phase d'hydrocarbures d'avec la partie vapeur de celle-ci dans une seconde zone de séparation (25), de fractionnement de la partie liquide de la phase d'hydrocarbures pour la séparation d'alkylat, de passage à travers un compresseur (34) et un condenseur (36) du produit en phase vapeur extrait dans la seconde zone de séparation, pour compresser et condenser la phase vapeur afin de former une phase liquide, de réduction de la pression exercée sur la phase liquide afin de la refroidir et de vaporiser des hydrocarbures volatils, pour la formation d'une phase liquide d'hydrocarbures isoparaffiniques et d'une phase vapeur d'hydrocarbures volatils, de séparation de la phase liquide d'hydrocarbures isoparaffiniques refroidie d'avec la phase vapeur d'hydrocarbures volatils dans une troisième zone de séparation, et d'addition de la phase liquide d'hydrocarbures isoparaffiniques refroidie séparée, en tant que corps réagissant dans la zone de réaction, caractérisé en ce que :

(a) l'appareil d'échange thermique (4) est muni d'une paroi conductrice de la chaleur avec une surface d'ébullition améliorée d'un côté de cette paroi pour effectuer l'échange thermique indirect entre la phase d'hydrocarbures refroidie et le mélange réactionnel, la phase d'hydrocarbures refroidie étant en contact avec la surface d'ébullition améliorée, au cours de l'échange thermique indirect ;

(b) on fait fonctionner le compresseur (34) sous une pression d'aspiration supérieure à la pression atmosphérique, laquelle pression d'aspiration est égale à la pression dans les seconde et troisième zones de séparation ; et

(c) la vitesse combinée d'alimentation en hydrocarbures isoparaffiniques et hydrocarbures oléfiniques de la zone de réaction est accrue d'une quantité égale à au moins 10% de la première vitesse d'alimentation, à une température de réaction qui est égale ou inférieure à ladite première température de réaction.

4. Procédé selon l'une quelconque des revendications 1 à 3, dans lequel la surface d'ébullition améliorée est une couche poreuse d'ébullition.

5. Procédé selon la revendication 4, dans lequel la couche poreuse d'ébullition est constituée de particules conductrices de la chaleur liées ensemble pour former des pores interconnectés de taille capillaire, ayant un rayon de pore équivalent inférieur à environ 0,15 mm (environ 6,0 millièmes de pouce).

6. Procédé selon la revendication 4, dans lequel la couche poreuse d'ébullition est formée par travail mécanique de la surface de la paroi.

7. Procédé selon l'une quelconque des revendications 1 à 6, dans lequel la surface d'ébullition améliorée donne un coefficient de transfert thermique dans la pellicule bouillante qui est supérieur à 2 271 W/m² K [400 (BTU/h)/(ft² °F)].

8. Procédé selon la revendication 7, dans lequel la couche poreuse d'ébullition donne un coefficient de transfert thermique dans la pellicule bouillante qui est supérieur à 5678 W/m² K [1000 (BTU/h)/(ft² °F)].

9. Procédé selon l'une quelconque des revendications 1 à 8, dans lequel la pression manométrique d'aspiration du compresseur est dans la plage de 0 à 48,3 kPa (0-7 psi).

10. Procédé selon la revendication 9, dans lequel la pression manométrique d'aspiration du compresseur est dans la plage de 13,8 à 27,6 kPa (2-4 psi).

11. Procédé selon l'une quelconque des revendications 1 à 10, dans lequel la différence de température entre la phase d'hydrocarbures refroidie et le mélange réactionnel au cours de l'échange thermique indirect est dans la plage de 5,6 à 11,1°C (10-20°F).

12. Procédé selon la revendication 11, dans lequel la différence de température est dans la plage de 5,6 à 8,3°C (10-15°F).

EP 0 281 411 B1

Propane and Lighter

Normal Butane

Rerun Light Alkylate

Alkylate Bottoms

Neutralization and Fractionation

Condenser

Compressor

Bleed Stream to Depropanizer

Depropanized IC₄ Recycle

Deisobutanizer Overhead

Isobutane Flash Drum

Isobutane Feed

Acid Settler

Isobutane Feed

Fresh Acid

Spent Acid

Olefinic Hydrocarbons

Pumping Impeller

Reactor

Driver

Suction Trap

Exchanger

Fig. 2

Fig. 1